# EUROPEAN PATENT APPLICATION

(11) **EP 2 839 816 A1**
(43) Date of publication of application: **25.02.2015**
(21) Application number: 13853318.7
(22) Date of filing: 07.11.2013
(51) Int. Cl.: A61F 5/44

(54) **DIAPER UNIT AND OUTER DIAPER WRAPPER**

(30) Priority: 08.11.2012 JP 2012245930
(71) Applicant: Unicare Corp., Bunkyo-ku, Tokyo 113-0001 (JP)
(72) Inventor: SASAKI Hidetada, Sendai-city Miyagi 982-0841 (JP); FUJII Masahiko, Sendai-city Miyagi 989-3214 (JP); KOMODA Masaji, Tokyo 113-0033 (JP)
(74) Representative: Kramer Barske Schmidtchen Patentanwälte PartG mbB
(86) International application number: PCT/JP2013/080090
(87) International publication number: WO 2014/073598

(57) **Abstract**

The present invention relates to a diaper unit comprising an outer diaper wrapper including an artificial gluteal block having a hole located substantially in the center, the artificial gluteal block being made up of an elastic member coming into contact with a gluteal region of a person, and a cover containing the artificial gluteal block and having a hole corresponding to the hole of the artificial gluteal block, the cover having a hook and loop fastener disposed at least on a portion of the cover facing a gluteal region of a person when being worn; and a inner diaper wrapper including a main diaper body portion having an opening in a feces receiving portion of a diaper and bonded to the outer diaper wrapper by a hook and loop fastener of the outer diaper wrapper, and a fecal bag coupled to the opening of the main diaper body and having a water-absorbing inner portion and a water-proof outer portion, the fecal bag being housed in the hole of the outer diaper wrapper.

## Description

### TECHNICAL FIELD

The present invention relates to a diaper unit particularly preferred for a person requiring care, and an outer diaper wrapper making up a portion of this diaper unit.

### BACKGROUND ART

As Japan is currently entering into an aging society, nursing care of elderly people is presenting a problem. Particularly, demand for diapers is increasing. In response, various types of diapers appear and the number of the types is estimated to reach as much as 1500. However, these various diapers are not necessarily satisfying in the present circumstances despite respective characteristics.

Particularly, it is problematic that loose feces adhere to a portion of a diaper facing the anus and go around from this portion to the back and the whole waist. This increases the risk of bacteria derived from feces entering a urinary tract (especially, female urinary tract) and causing urinary tract infection leading to inflammation of the bladder. In the case of bedridden patients, filth adheres to a bedsore in a sacrum portion and gives no healing time to the bedsore, rather increasing the risk of worsening of symptoms. These infections are widely treated by using antibiotics in general. Excessive use of the antibiotics may cause methicillin-resistant Staphylococcus aureus (MRSA) infection etc. Because of scarcity of effective antibiotics, this infection may possibly cause nosocomial infection and may result in an extremely serious state. Under such circumstances, it is urgently necessary to prevent the occurrence of the infection for patients (particularly elderly people) with weakened immunity. However, currently commercially available diapers cannot prevent feces from scattering, which is extremely serious problem.

A human body has a sacrum between left and right gluteus maximus muscles and, as the gluteus maximus muscles decrease, the sacrum is projected outward from a gluteal region. If a parson in this state is lying in a supine position, the sacrum is pressed and compressed against bedding by a body weight. The weights applied to parts of a person in a lying state are considered as follows. When a person is lying straight in a supine position, about a half of the body weight is applied to a waist portion. For example, in the case of a person weighing 50 kg, this corresponds to a value of approx. 25 Kg. Therefore, a tip portion of the sacrum has poor blood flow. The poor blood flow prevents leucocytes included in blood from arriving, weakening a defense function against bacteria.

The anus is present on the lower side of the sacrum and feces excreted from the anus are likely to adhere to the sacrum, especially in the case of a bedridden person. Feces contain a large amount of bacteria such as Escherichia coli and it is also known that feces themselves are strong alkaline. Therefore, feces adhering to skin cause strong itchiness. The skin around the sacrum is damaged and broken by scratching when the itchiness is caused or by friction with bedding while a person is lying down, and bacteria grow due to adherence of feces, in a repeated manner, resulting in a bedsore (decubitus).

It is considered that seventy percent of the bedsores developed in bedridden people occurs in the skin around the sacrum. Many bedridden people such as elderly people use diapers. A diaper used in this case is set such that the diaper is wrapped around the entire buttocks. The diaper is configured to directly receive feces excreted from the anus with a water absorbing layer containing a polymer regardless of whether the diaper is of a flat-type or a pants-type. Therefore, the feces go around to the back in the case of elderly people with decreased gluteus maximus muscles. Especially, as time elapses from defecation, feces spread to portions in the diaper due to body movement. This state is a major problem for a person with a bedsore.

If a bedsore is already developed in a portion around the sacrum, feces go around to the affected area as described above and the affected area is contaminated. Even if the affected area is covered by a therapeutic product such as gauze and adhesive plaster for treatment, the therapeutic product is contaminated. Therefore, the affected area is contaminated each time a person defecates and therefore is not cured, and replacement of the therapeutic product and disinfection are required. The disinfection also stimulates the affected area and causes a delay in healing.

Another major problem is to reduce/limit a range contaminated by feces to reduce physical/mental burdens of a caregiver and a care receiver.

A diaper cover type bedpan is known that is basically configured in the same way as a normal diaper cover and that has the following features in terms of reduction of the burden of a caregiver and economic efficiency. The diaper cover type bedpan includes a bedpan having a trumpet-shaped urination outflow cylinder opened at a tip in an urination/defecation part and a pants-shaped bedpan cover disposed to cover the bedpan. The bedpan cover of the diaper cover type bedpan further includes an elongated bottomed cylindrical urine collection member communicating with the urination outflow cylinder and receiving and storing urine and feces (see Patent Document 1).

An excretion time actuation mechanism is known that has structure disposed with a slit in an urination/defecation part of a diaper and provided with a bag-like receiving space for excreta on the outside of the slit (see Patent Document 2). In this excretion time actuation mechanism, air is sent at the time of excretion to inflate the bag-like receiving space and the slit disposed in the urination/defecation part of the diaper is opened to form an opening.

The diaper cover type bedpan necessitates a formation of a gap for setting the urine collection member with a mattress etc., and has a problem of troublesome and burdensome work. It is also disadvantageous that movement of a person wearing the diaper cover type bedpan changes the shape of the gap and prevents proper excretion to the urine collection member. In this regard, although the excretion time actuation mechanism eliminates the need for preparation of a mattress etc., air must be sent at the time of excretion and, therefore, the mechanism has a problem of a large-scale configuration resulting in a cost increase.

### PRIOR ART DOCUMENT

### Patent Documents

Patent Document 1: Specification of Japanese Utility Model Registration no. 2535259
Patent Document 2: Japanese Laid-Open Patent Publication No. 2009-119220

### DISCLOSURE OF THE INVENTION

### Problem to Be Solved by the Invention

The present invention was conceived to solve the problems related to conventional urination/defecation treatment as described above. It is therefore an object of the present invention to provide a diaper unit and an outer diaper wrapper that eliminate the need for a complicated high-cost configuration such as those timely sending air at the time of excretion, that can catch excreta regardless of whether a person is sitting or supine and substantially independent of the person's body movement, and that are sanitary and capable of reducing the burden of a caregiver. Means for Solving the Problem

The diaper unit according to the present invention includes
an outer diaper wrapper including an artificial gluteal block having a hole located substantially in the center, the artificial gluteal block being made up of an elastic member coming into contact with a gluteal region of a person, and a cover containing the artificial gluteal block and having a hole corresponding to the hole of the artificial gluteal block, the cover having a hook and loop fastener disposed at least on a portion of the cover facing a gluteal region of a person when being worn; and
a inner diaper wrapper including a main diaper body portion having an opening in a feces receiving portion of a diaper and bonded to the outer diaper wrapper by a hook and loop fastener of the outer diaper wrapper, and a fecal bag coupled to the opening of the main diaper body and having a water-absorbing inner portion and a water-proof outer portion, the fecal bag being housed in the hole of the outer diaper wrapper.

The diaper unit according to the present invention is characterized in that a bottom portion of the hole of the outer diaper wrapper is closed by the cover, and wherein a hook and loop fastener is disposed on the bottom portion of the hole.

The diaper unit according to the present invention is characterized in that an impact absorbing gel piece is disposed between the cover and the artificial gluteal block.

The diaper unit according to the present invention is characterized in that a concave portion is formed in a part of the artificial gluteal block coming into contact with the sacrum/coccyx such that a downslope is formed toward the outside.

The diaper unit according to the present invention is characterized in that a downslope is formed toward the outside in a part of the artificial gluteal block coming into contact with a boundary portion between the gluteal region and legs.

The diaper unit according to the present invention is characterized in that foamed resin is disposed in a part of the artificial gluteal block coming into contact with gluteal muscle, wherein the foamed resin has elasticity different from a member making up a major portion of the artificial gluteal block and has a shape convexed toward gluteal muscle.

The diaper unit according to the present invention is characterized in that the fecal bag is made up of an inner bag containing a high-molecular absorbing polymer and a moisture-permeable waterproof outer bag.

The diaper unit according to the present invention is characterized in that an opening of the outer bag of the fecal bag is coupled to a peripheral edge of the opening in the main diaper body portion by thermocompression bonding or adhesive bonding.

The outer diaper wrapper according to the present invention includes an artificial gluteal block having a hole located substantially in the center, the artificial gluteal block being made up of an elastic member coming into contact with a gluteal region of a person; and a cover containing the artificial gluteal block and having a hole corresponding to the hole of the artificial gluteal block, the cover having a hook and loop fastener disposed at least on a portion of the cover facing a gluteal region of a person when being worn.

The outer diaper wrapper according to the present invention is characterized in that a bottom portion of the hole of the outer diaper wrapper is closed by the cover, and wherein a hook and loop fastener is disposed on the bottom portion of the hole.

The outer diaper wrapper according to the present invention is characterized in that an impact absorbing gel piece is disposed between the cover and the artificial gluteal block.

### Effect of the Invention

Since the main diaper body portion of the inner diaper wrapper having the fecal bag in bonded to the outer diaper wrapper by hook and loop fasteners of the outer diaper wrapper, the diaper unit and the outer diaper wrapper according to the present invention can catch excreta regardless of whether a person is sitting or supine and substantially independent of the person's body movement without displacement of the inner diaper wrapper. Therefore, usage of the diaper unit and the outer diaper wrapper according to the present invention hardly allows feces to go around to the back and is sanitary and capable of reducing the burden of a caregiver. The diaper unit and the outer diaper wrapper according to the present invention can advantageously be realized at lower cost because of less complicated configuration. The effects can further be increased by disposing the hook and loop fasteners on the bottomed portion in the hole of the outer diaper wrapper.

Since the impact absorbing piece is disposed between the cover and the artificial gluteal block, the diaper unit and the outer diaper wrapper according to the present invention distribute the stress applied to the gluteal region and hardly cause a bedsore in the sacrum portion, and have the effect of suppressing deterioration of a bedsore if the bedsore has been developed.

Since the concave portion is formed in the part of the artificial gluteal block coming into contact with the sacrum/coccyx and the downslope is formed toward the outside, the diaper unit according to the present invention can reduce a possibility of the sacrum/coccyx receiving pressure from the outer diaper wrapper and can make it difficult to develop a bedsore in the sacrum/coccyx portion. Since the diaper unit according to the present invention has the downslope formed toward the outside in the part of the artificial gluteal block coming into contact with the boundary portion between the gluteal region and the legs, the diaper unit properly fits the gluteal region of the body as a whole and can catch excreta even while a person is lying down or sitting.

Since the foamed resin having elasticity different from a member making up a major portion of the artificial gluteal block is disposed convexly toward the gluteal muscle in the part of the artificial gluteal block coming into contact with the gluteal muscle, the diaper unit according to the present invention acts as artificial gluteal muscle for an elderly person with the gluteal muscle reduced. As a result, the pressure applied to the sacrum/coccyx from the outer diaper wrapper can be reduced to make it difficult to develop a bedsore in the sacrum/coccyx portion.

In the diaper unit according to the present invention, the opening of the outer bag of the fecal bag is coupled to the peripheral edge of the opening in the main diaper body portion by thermocompression bonding or adhesive bonding and, therefore, the inner diaper wrapper can be created by forming an opening in a normal diaper and coupling the fecal bag. As a result, the diaper unit according to the present invention can inexpensively and easily be created. Since the fecal bag is made up of the inner bag containing a polymer and the moisture-permeable waterproof outer bag, the diaper unit according to the present invention hardly becomes stuffy and can adequately prevent leakage of feces.

### BRIEF DESCRIPTION OF THE DRAWINGS

[Fig. 1] Fig. 1 is a perspective view of an embodiment of a diaper unit according to the present invention.
[Fig. 2] Fig. 2 is a perspective view of an outer diaper wrapper making up the embodiment of the diaper unit according to the present invention with an inside artificial gluteal block removed.
[Fig. 3] Fig. 3 is an assembly perspective view of a first embodiment of the artificial gluteal block contained in the outer diaper wrapper making up the embodiment of the diaper unit according to the present invention.
[Fig. 4] Fig. 4 is a perspective view of the outer diaper wrapper making up the embodiment of the diaper unit according to the present invention viewed from the inside.
[Fig. 5] Fig. 5 is a perspective view of the outer diaper wrapper making up the embodiment of the diaper unit according to the present invention viewed from the outside.
[Fig. 6] Fig. 6 is a perspective view of a inner diaper wrapper making up the embodiment of the diaper unit according to the present invention viewed from the inside.
[Fig. 7] Fig. 7 is a perspective view of the inner diaper wrapper making up the embodiment of the diaper unit according to the present invention viewed from the outside.
[Fig. 8] Fig. 8 is a partially cutaway perspective view of a fecal bag used in the inner diaper wrapper making up the embodiment of the diaper unit according to the present invention.
[Fig. 9] Fig. 9 is a side view of a usage state of the embodiment of the diaper unit according to the present invention.
[Fig. 10] Fig. 10 is an assembly perspective view of a second embodiment of the artificial gluteal block contained in the outer diaper wrapper making up the embodiment of the diaper unit according to the present invention.
[Fig. 11] Fig. 11 is a perspective view of a complete state of the second embodiment of the artificial gluteal block contained in the outer diaper wrapper making up the embodiment of the diaper unit according to the present invention.
[Fig. 12] Fig. 12 is a cross-sectional view taken along a line A-A of Fig. 11.

### MODE FOR CARRYING OUT THE INVENTION

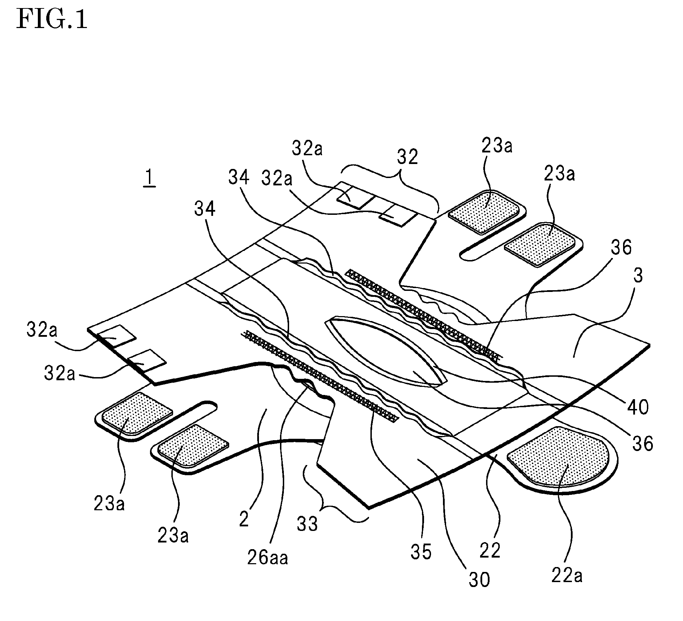

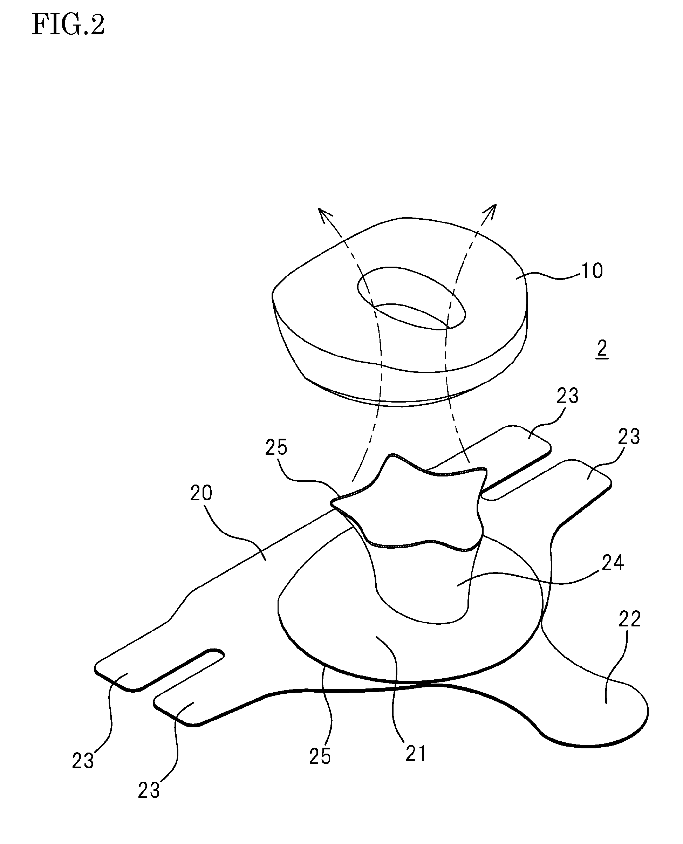

Embodiments of a diaper unit and an outer diaper wrapper of the present invention will now be described with reference to the accompanying drawings. In the figures, the same constituent elements are denoted by the same reference numerals and will not repeatedly be described. As depicted in Fig. 1, a diaper unit 1 is formed by overlapping a inner diaper wrapper 3 on the inside of an outer diaper wrapper 2. As depicted in Fig. 2, the outer diaper wrapper 2 is formed by wrapping an artificial gluteal block 10 with a cover 20.

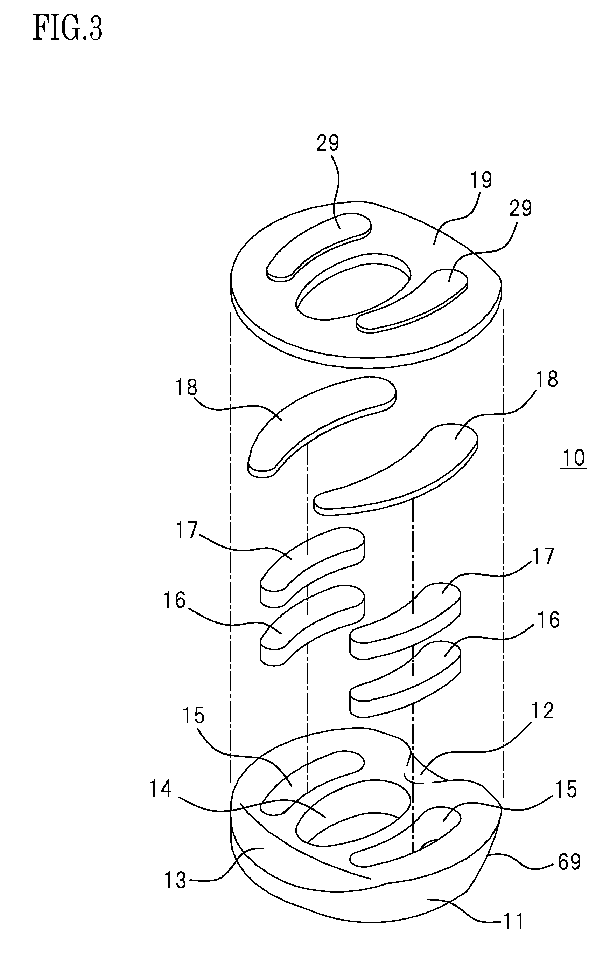

As depicted in Fig. 3, the artificial gluteal block 10 includes a base block 11 mainly made up of an elastic member formed in an annular shape surrounding a gluteal region of a person. The base block 11 is the elastic member coming into indirect contact with the gluteal region of a person. Although the outer shape of the base block 11 is preferably a circular annular shape, this is not a limitation and an annular outer edge may be in a rectangle shape, a polygonal shape, etc. The base block 11 can be made of semi-rigid urethane foam. Although a thickness of the base block 11 is preferably 25 to 55 mm thick while a wearer is applying a body weight, this is not a limitation. A surface of the base block 11 facing the gluteal region has a U- or V-shaped concave portion 12 formed in a part corresponding to the sacrum/coccyx when being worn. The concave portion 12 has a downslope formed toward the outside of the circular annular portion. As a result, a possibility of the sacrum/coccyx receiving pressure from the outer diaper wrapper 2 can be reduced to make it difficult to develop a bedsore in the sacrum/coccyx portion.

The surface of the base block 11 facing the gluteal region has a downward sloped surface 13 formed toward the outside of the circular annular portion in a part corresponding to a boundary portion between the gluteal region and the legs when being worn. The sloped surface 13 has a longest slope face at a center portion thereof and is formed such that a length of the slope face is made shorter toward the both end portions. The sloped surface 13 allows the diaper unit 1 to suitably fit the gluteal region of the body as a whole so as to catch excreta even while a person is lying down or sitting. A sloped surface 69 is also formed on the back surface side of the surface provided with the concave portion 12. The sloped surface 69 is formed such that a thickness of the base block 11 increases from the back surface side of a position at which the concave portion 12 is defined as an outer peripheral edge of the base block 11, toward a hole 14 in a substantially center portion of the base block 11. The sloped surface 69 makes a line from the back of the wearer to the outer diaper wrapper 2 natural and can achieve a favorable wearing feeling while the wearer is lying down.

The hole 14 in the center portion of the base block 11 can be in an oval shape of approx. 150 mm in the major axis and about 100 mm in the minor axis. The major axis and the minor axis are changed as needed depending on a body shape of a person. Peripheral edges of an entry and an exit of the hole 14 can be in a chamfered configuration. A shape of the hole 14 is not limited to a cylindrical shape and may be a prismatic shape, an elliptic cylindrical shape, etc., and particularly, a plane shape of a wall surface of the hole 14 may be any appropriate shape. Shapes of a hole 26 of the cover 20 and an opening 36 of a main diaper body portion 30 described later are changed depending on a shape of the hole 14.

Foamed resin is disposed symmetrically and convexly toward the gluteal muscle in the parts corresponding to the gluteal muscle (on which the gluteal muscle is placed) when the base block 11 is worn. The symmetrically foamed resin has elasticity different from a member making up a major portion of the artificial gluteal block 10. In particular, holes 15 formed into a circular arc plate shape (in a shape of broad beans in this embodiment) are made in the parts corresponding to the gluteal muscle (on which the gluteal muscle is placed) when the base block 11 is worn. Bottom plate pieces 16 having the same shape as the holes 15 are embedded into bottom portions of the holes 15. The bottom plate pieces 16 can be made of the same anti-rigid urethane foam as the base block 11. The bottom plate pieces 16 may have a thickness that is about half the depth of the holes 15. When the holes 15 are made, parts corresponding to the bottom plate pieces 16 can be left to omit the embedment of the bottom plate pieces 16.

Intermediate pieces 17 having the same shape as the bottom plate pieces 16 and a thickness slightly larger than the bottom plate pieces 16 are embedded on the bottom plate pieces 16. The intermediate pieces 17 can be made of general-purpose urethane foam. Upper plate pieces 18 having a thickness equal to about half the thickness of the intermediate pieces 17 are placed on the intermediate pieces 17. The upper plate pieces 18 have a shape acquired by enlarging the plane shape of the intermediate pieces 17 and can be made of low-resilient urethane foam.

"Anti-rigidity", "general-purpose", and "low-resilience" of the urethane foam described above are used for relative classification. For example, in the classification according to impact resilience of the JIS standard K-6400-3, the "anti-rigid urethane foam", "the general-purpose urethane foam", and the "low-resilient urethane foam" may be referred to as "high elasticity foam", "general foam", and "low impact resilience foam", respectively. By way of example, the "anti-rigid urethane foam" may have density of 40±2 (Kg/m³) and impact resilience of 60 or higher; the "general-purpose urethane foam" may have density of 48±3 (Kg/m³) and impact resilience of 40 to 50; and the "low-resilient urethane foam" may have density of 22±2 (Kg/m³) and impact resilience of 2 to 6.

The intermediate pieces 17 are partially projected from the holes 15 and are covered by the upper plate pieces 18. The intermediate pieces 17 and the upper plate pieces 18 are coupled by adhesive bonding, for example, and the intermediate pieces 17 are fixed in the holes 15. A covering sheet 19 is disposed thereon that has substantially the same shape as the surface shape of the base block 11 and a thickness of approx. 5 to 10 mm. The covering sheet 19 can be made of the general urethane foam. The covering sheet 19 is placed in a state of covering the surface of the base block 11 with the upper plate pieces 18, the intermediate pieces 17, and the bottom plate pieces 16 lying thereunder. The peripheral edge portion of the covering sheet 19 is fixed in this state to the peripheral edge portion of the base block 11 by adhesive bonding etc.

As described above, the foamed resin such as urethane is disposed convexly toward the gluteal muscle in the parts of the artificial gluteal block 10 corresponding to the gluteal muscle when being worn. The elasticity of the foamed resin can be set to elasticity different from a member making up a major portion of the artificial gluteal block 10. Impact absorbing gel pieces 29 are affixed to positions corresponding to the intermediate pieces 17 and the bottom plate pieces 16 on the surface of the covering sheet 19. The impact absorbing gel pieces 29 are plate bodies having the same plane shape as the intermediate pieces 17 and the bottom plate pieces 16 and can be those having impact absorbing gel enclosed by a sheet. An elastic member including the covering sheet 19, the intermediate pieces 17, the bottom plate pieces 16, the upper plate pieces 18, and the impact absorbing gel pieces 29 comes into contact with the ischium (a bone located in a rear portion of the coxal bone and supporting the trunk in a sitting position) to support the body weight while the diaper unit is worn. The elastic member can achieve a comfortable wearing feeling such that a thin elderly person hardly feels pain in the gluteal region in a sitting position. The elastic member including the covering sheet 19, the intermediate pieces 17, the bottom plate pieces 16, the upper plate pieces 18, and the impact absorbing gel pieces 29 may be made of the same elastic member as a whole. The base block 11 and the elastic member can be created by molding.

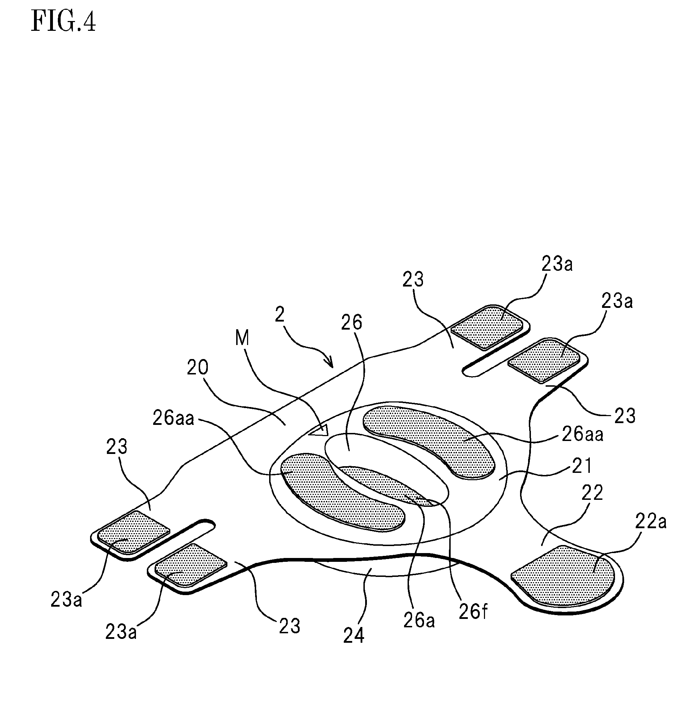

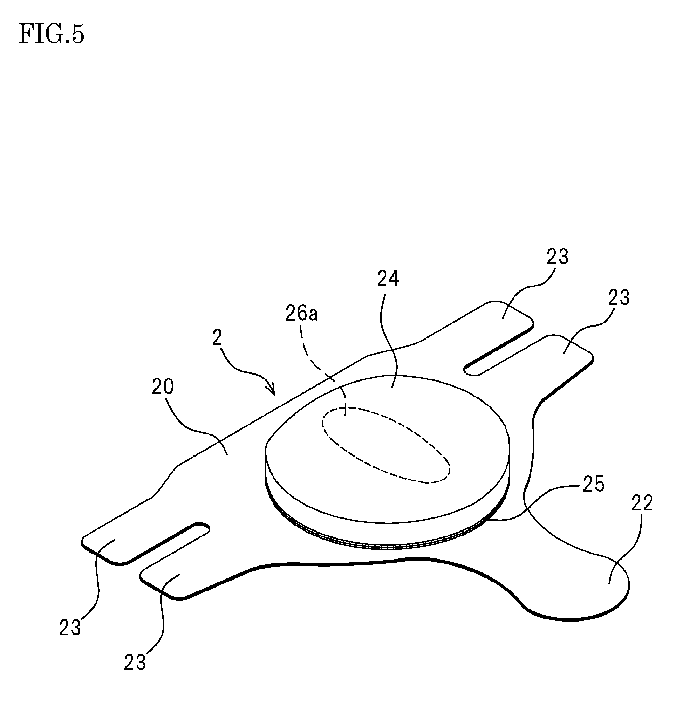

The cover 20 has a diaper cover shape as depicted in Figs. 4 and 5. In particular, the cover 20 has a front standing portion 22 extended in a band shape from a substantially trapezoidal center portion 21 on which the gluteal region is placed, toward the side located forward when being worn. The cover 20 also has two belt-like waist wrapping portions 23 extended from the center portion 21 toward each of the sides corresponding to left and right waist portions when being worn. The cover 20 can be made of a composite material created by attaching pieces of fabric such as knitted fabric to front and back surfaces of compressed urethane.

A hook and loop fastener 22a is disposed on an inner tip of the front standing portion 22, and hook and loop fasteners 23a are disposed on inner tips of the waist wrapping portions 23. By disposing a sacrum portion mark M (Fig. 4) at a surface position of the cover 20 covering the part of the U- or V-shaped concave portion 12 of the base block 11, the sacrum portion mark M can be used as a mark for wearing.

A bag portion 24 housing the artificial gluteal block 10 is disposed on the back surface side (front surface when being worn) of the center portion 21 of the cover 20. A fastener 25 is substantially circumferentially disposed in a shape along a peripheral edge portion of the artificial gluteal block 10 on the back surface side of the center portion 21 of the cover 20 and the bag portion 24 is detachably disposed on the backside of the center portion 21 of the cover 20. Therefore, while an opening of the bag portion 24 is opened by the fastener 25, the artificial gluteal block 10 can be taken out as depicted in Fig. 2.

The bag portion 24 includes the hole 26 corresponding to the hole 14 of the center portion in the circular annular portion of the artificial gluteal block 10 and the hole 26 has a depth from the front surface side to the back surface of the center portion 21 of the cover 20. In other words, a wall surface of the hole 14 is covered by a sheet of the hole 26 of the cover 20. The hole 26 of the cover 20 is bottomed and a hook and loop fastener 26a can be disposed on a bottom surface 26f of the hole 26. A hook and loop fastener may also be disposed on a wall surface of the hole 26. Hook and loop fasteners 26aa having a circular arc plate shape are disposed around the hole 26 of the cover 20 in at least two parts of the cover 20 facing the gluteal region of a person when being worn.

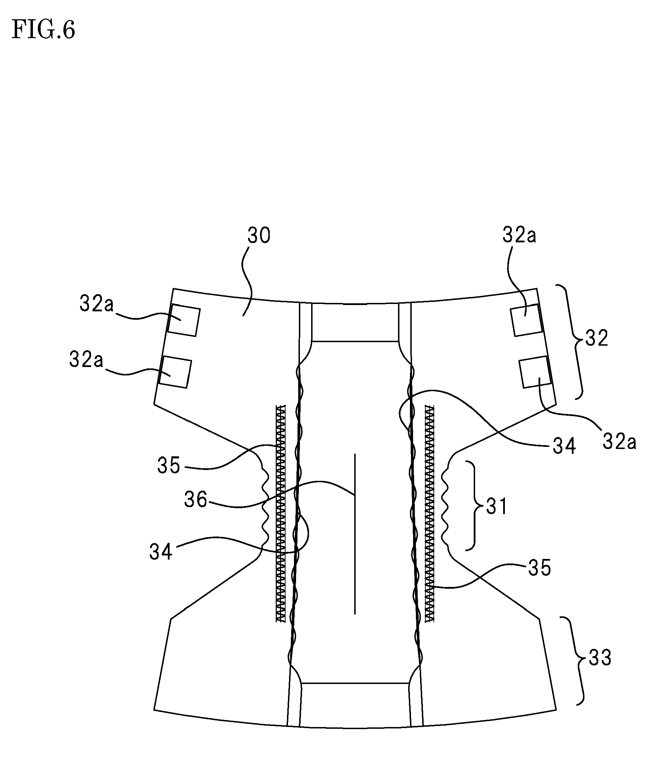

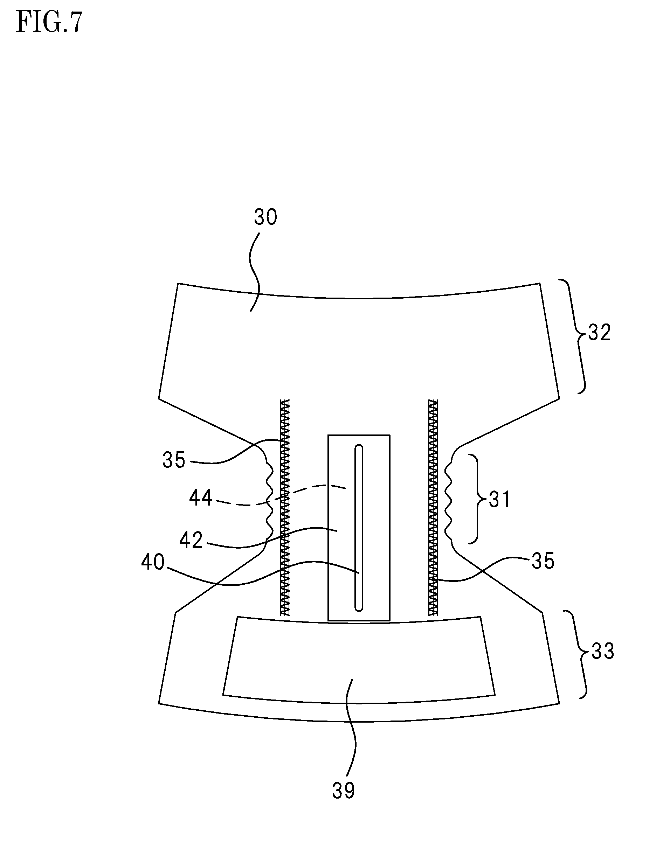

As depicted in Figs. 6 and 7, the inner diaper wrapper 3 includes the main diaper body portion 30 that is a paper diaper made up of a feces receiving portion 31 that is a rectangular region in a center portion, a back side covering portion 32 in a substantially trapezoidal shape, and an belly side covering portion 33 in a substantially trapezoidal shape. The back side covering portion 32 is integrally formed on one end side of the feces receiving portion 31 in the center portion, and the belly side covering portion 33 is integrally formed on the other end of the feces receiving portion 31.

The inner side (the side coming into contact with a person) of the main diaper body portion 30 is made up of a water-absorbing sheet containing a high-molecular absorbing polymer and the outer side is preferably made up of a moisture-permeable waterproof sheet made of paper fiber. One or more layers of water-absorbing members continued to the water-absorbing sheet are interposed between the water-absorbing sheet and the moisture-permeable waterproof sheet. A urinary leakage preventing gathers 34 are implanted in the both side edge portions of the feces receiving portion 31. The gathers 34 is extend to regions of the back side covering portion 32 and the belly side covering portion 33 of the main diaper body portion 30. Rubber strings 35 are sewn on the outside of the gathers 34 along the gathers 34 with a dimension shorter than the gathers 34. The rubber strings 35 allows expansion and contraction of the parts of the main diaper body portion 30 on which the rubber strings 35 are sewn, and this portions fit the legs near the bases thereof.

Folded hook and loop fasteners 32a are disposed on a lateral side end portion of the back side covering portion 32. The hook and loop fasteners 32a are stretched from the folded state into a band shape in use, and the band-like hook and loop fasteners 32a are bonded to a lateral side end portion 39 of the belly side covering portion 33. As a result, the back side covering portion 32 and the belly side covering portion 33 are coupled into a form of a pants-type diaper. The lateral side end portion 39 may be made up of a sheet subjected to an adhesion reinforcing process for strong adhesion of the hook and loop fasteners 32a.

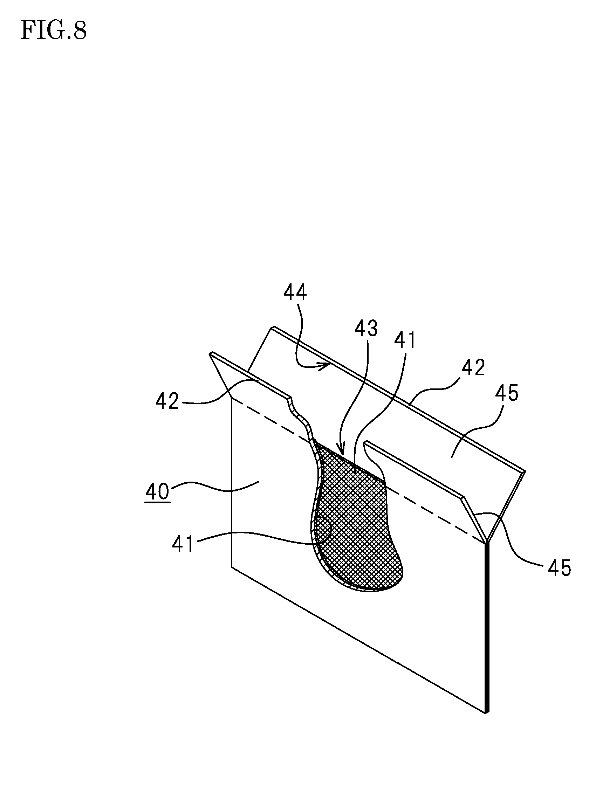

A lien of the opening 36 is formed in the center of the feces receiving portion 31, extending in directions to the back side covering portion 32 and the belly side covering portion 33. The peripheral edge of the opening 36 is sealed by the ultrasonic cutting processing or the emboss processing to prevent protrusion of a member present between the water-absorbing sheet and the moisture-permeable waterproof sheet in the main diaper body portion 30. The opening 36 is coupled to a fecal bag 40 including an inner bag 41 and an outer bag 42 depicted in Fig. 8. The inner bag 41 of the fecal bag 40 is made of paper fiber (pulp) and the inner bag 41 is made up of a water-absorbing mesh sheet containing a high-molecular absorbing polymer. A moisture-permeable non-woven fabric is disposed on a surface of the inner bag 41 coming into contact with feces. The outer bag 42 of the fecal bag 40 can preferably be made up of a moisture-permeable waterproof sheet. A non-woven fabric is affixed to an exposed surface of the outer bag 42 of the fecal bag 40. This non-woven fabric includes a surface firmly attached to the hook and loop fastener 26a disposed in the hole 26 of the inner diaper wrapper 3 and, therefore, the fecal bag 40 is properly fixed in the hole 26 of the outer diaper wrapper 2.

The configuration of the fecal bag is not limited to the configuration described above. For example, the outer bag 42 may have the outer bag configuration described above and the inner bag may be made up of a water-absorbing sheet containing a high-molecular absorbing polymer such that a portion of the water-absorbing mesh sheet containing a high-molecular absorbing polymer made of paper fiber (pulp) is attached to an inner side of the inner bag (inner wall side of the inner bag) or an outer side thereof (outer wall side of the inner bag). The fecal bag may be a fecal bag described in Japanese Patent Application No. 2012-139095 filed by the applicants of the present application.

An opening end portion 43 of the inner bag 41 is located at a position lower than an opening end portion 44 of the outer bag 42. An opening inner side region 45 of the outer bag 42 is a region for thermocompression bonding or adhesive bonding. The opening inner side region 45 of the outer bag 42 is thermocompression-bonded or adhesively bonded to an edge portion of an outer side surface (back surface) at the opening 36 of the feces receiving portion 31 and the fecal bag 40 is coupled as depicted in Fig. 7. The inner bag 41 of the fecal bag 40 and an inner surface in the opening 36 of the feces receiving portion 31 can be coupled by adhesive tape etc.

The diaper unit 1 can be made up by using the inner diaper wrapper 3 and the outer diaper wrapper 2 configured as described above. To place the diaper unit 1 in a usable state, the fecal bag 40 is expanded into a cylindrical state by putting a hand into the opening 36 of the inner diaper wrapper 3, and the fecal bag 40 in a cylindrical state is inserted into the hole 26 of the outer diaper wrapper 2 such that a bottom portion of the fecal bag 40 is firmly coupled to the hook and loop fastener 26a disposed in the hole 26. A periphery of the opening 36 in the inner diaper wrapper 3 is coupled to the hook and loop fasteners 26aa of the outer diaper wrapper 2. This state is the state of Fig. 1.

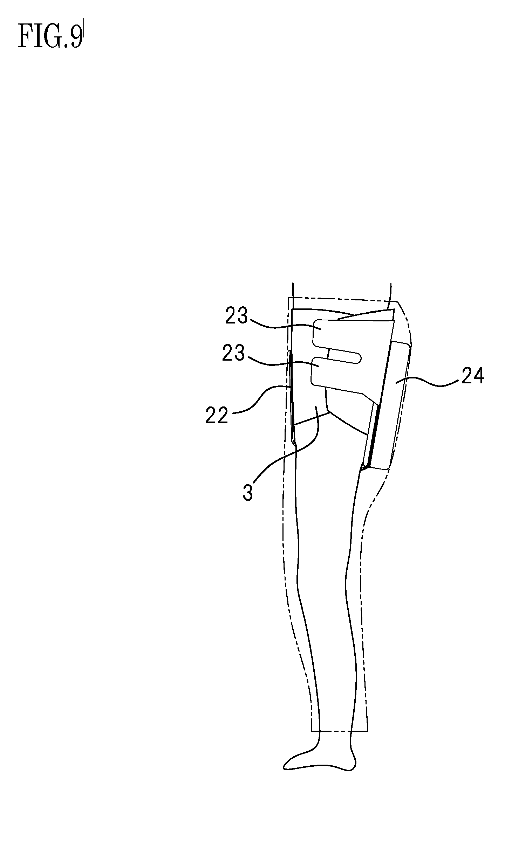

The diaper unit 1 assembled in this way is pushed in the gluteal region of a person requiring care etc., such that the center of the opening 36 is located at a position corresponding to the anus, and the inner diaper wrapper 3 is worn by the person in the same way as a diaper. The front standing portion 22 of the outer diaper wrapper 2 is folded toward the belly side, and the hook and loop fastener 22a is pushed against an outer surface of the belly side covering portion 33 of the inner diaper wrapper 3 to couple the outer diaper wrapper 2 to the inner diaper wrapper 3. The belt-like waist wrapping portions 23 are pulled out toward the belly side of the person, and the hook and loop fasteners 23a are pushed against the waist side to couple the outer diaper wrapper 2 to the inner diaper wrapper 3. This stat is depicted in Fig. 9.

In the diaper unit 1 described above, the main diaper body portion of the inner diaper wrapper 3 having the fecal bag 40 is bonded to the outer diaper wrapper 2 by the hook and loop fasteners 26aa of the outer diaper wrapper 2. Therefore, the inner diaper wrapper 3 and the outer diaper wrapper 2 are not shifted from each other in the coupled state. The diaper unit 1 can catch excreta regardless of whether a person is sitting or supine and substantially independent of the person's body movement. Therefore, the diaper unit 1 hardly allows feces to go around to the back and is sanitary and capable of reducing the burden of a caregiver. In the diaper unit 1, the fecal bag 40 is fixed to the hook and loop fastener 26a in an expanded state in the hole 26 of the outer diaper wrapper 2. Therefore, since feces excreted in the fecal bag 40 can be retained, feces are prevented from attaching to the sacrum and a range contaminated by feces can be reduced/limited. Therefore, the diaper unit 1 can reduce physical/mental loads of a caregiver and a care receiver.

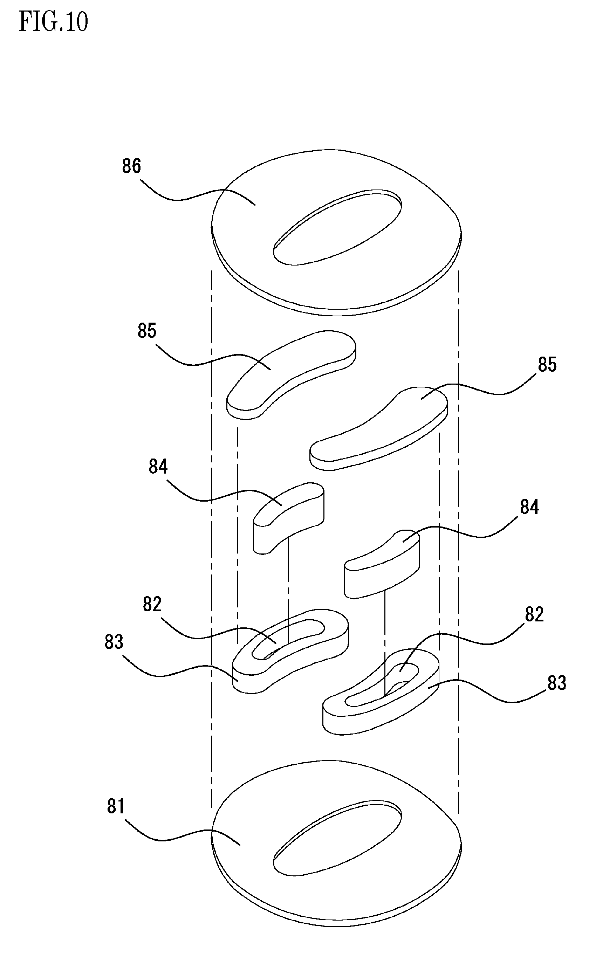

An artificial gluteal block 80 capable of substituting for the artificial gluteal block 10 described in Fig. 3 can be created from members as depicted in Fig. 10. The artificial gluteal block 80 includes a circular annular base sheet 81, intermediate blocks 83 having holes 82, core blocks 84 embedded in the holes 82, lid blocks 85 for covering the intermediate blocks 83 and the core blocks 84, and a disk-shaped surface sheet 86 positioned on the surface side.

The circular annular base sheet 81 can be made of polyethylene foam with a thickness of approx. 10 mm, for example. The intermediate blocks 83 can be made up of a bonded product of crushed foam referred to as chip urethane etc., with a thickness of approx. 20 mm, for example. The core blocks 84 can be made of general-purpose urethane foam with a thickness of approx. 40 mm, for example. The lid blocks 85 can be made of low-resilient urethane foam with a thickness of approx. 20 mm, for example. The surface sheet 86 can be made of soft polyurethane foam with a thickness of approx. 20 mm, for example. The soft polyurethane foam refers to polyurethane foam widely used in chairs and mattresses as well as sponge scrubbers.

The intermediate blocks 83 and the holes 82 have a broad bean shape. The intermediate blocks 83 are symmetrically disposed at positions of the gluteal region placed on the base sheet 81. The intermediate block 83 is bonded to the base sheet 81. The core blocks 84 are embedded in the holes 82 of the intermediate blocks 83 and bonded to the intermediate blocks 83. The core blocks 84 and the intermediate blocks 83 are covered by the lid blocks 85 from the upper side to bond the core blocks 84 and the intermediate blocks 83 to the lid blocks 85.

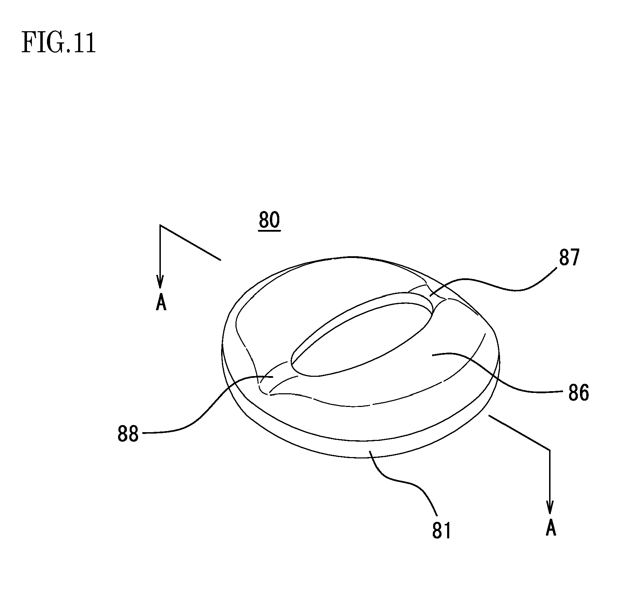

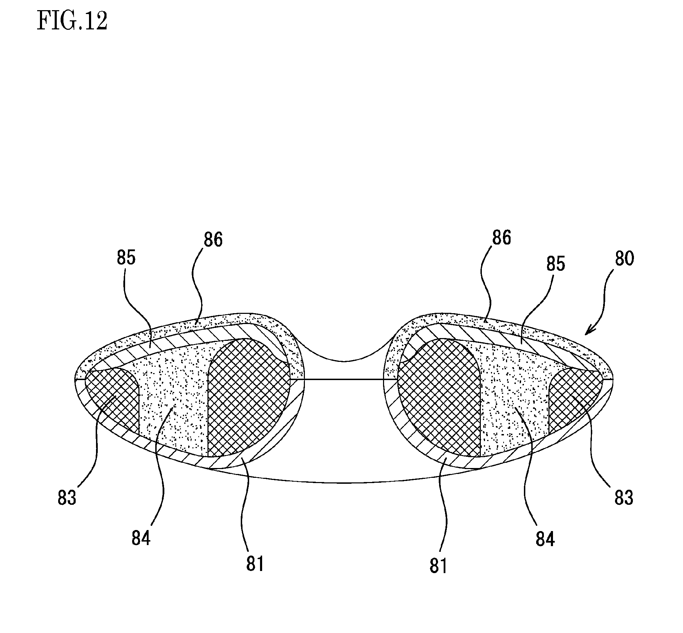

While the intermediate blocks 83, the core blocks 84, and the lid blocks 85 are disposed on the base sheet 81, the surface sheet 86 is put on these members. A peripheral edge portion of the base sheet 81 and a peripheral edge portion of the surface sheet 86 are pulled to overlap and bond the respective peripheral edge portions with each other. An edge portion of the base sheet 81 facing the hole in the center portion and an edge portion of the surface sheet 86 facing the hole in the center portion are pulled to overlap and bond the respective edge portions with each other. As a result, concave portions 87 and 88 are generated in the surface sheet 86 at the parts without the intermediate blocks 83 and the artificial gluteal block 80 is completed. Fig. 11 is a perspective view of the complete artificial gluteal block 80 and Fig. 12 is a cross-sectional view taken along a line A-A of Fig. 11. The concave portion 87 corresponds to the concave portion 12 of the artificial gluteal block 10 of Fig. 2. The concave portion 88 corresponds to the sloped portion 13 of the artificial gluteal block 10 of Fig. 2. The artificial gluteal block 80 can produce the same effects as the artificial gluteal block 10. A pair of the impact absorbing gel pieces 29 may be affixed onto the surface sheet 86 as depicted in Fig. 3.

Although both the artificial gluteal block 10 and the artificial gluteal block 80 have a circular annular shape, the positions of the sloped portion 13 and the concave portion 88 may be cut out to form a horseshoe shape. In the artificial gluteal block 10 and the artificial gluteal block 80, respective impact absorbing gel pieces may be disposed on the covering sheet 19 above the concave portion 12 and the surface sheet 86 above the concave portion 87 in addition to the pair of the impact absorbing gel pieces 29. An impact absorbing gel piece may be disposed only on the covering sheet 19 above the concave portion 12 or only on the surface sheet 19 above the concave portion 87.

### EXPLANATIONS OF LETTERS OR NUMERALS

- 1: diaper unit
- 2: outer diaper wrapper
- 3: inner diaper wrapper
- 10: artificial gluteal block
- 11: base block
- 20: cover
- 24: bag portion
- 26: hole
- 29: impact absorbing gel piece
- 31: feces receiving portion
- 32: back side covering portion
- 33: belly side covering portion
- 40: fecal bag
- 41: inner bag
- 42: outer bag

## Claims

1. A diaper unit comprising:
an outer diaper wrapper including
an artificial gluteal block having a hole located substantially in the center, the artificial gluteal block being made up of an elastic member coming into contact with a gluteal region of a person, and
a cover containing the artificial gluteal block and having a hole corresponding to the hole of the artificial gluteal block, the cover having a hook and loop fastener disposed at least on a portion of the cover facing a gluteal region of a person when being worn; and
a inner diaper wrapper including
a main diaper body portion having an opening in a feces receiving portion of a diaper and bonded to the outer diaper wrapper by a hook and loop fastener of the outer diaper wrapper, and
a fecal bag coupled to the opening of the main diaper body and having a water-absorbing inner portion and a water-proof outer portion, the fecal bag being housed in the hole of the outer diaper wrapper.

2. The diaper unit of claim 1, wherein a bottom portion of the hole of the outer diaper wrapper is closed by the cover, and wherein a hook and loop fastener is disposed on the bottom portion of the hole.

3. The diaper unit of claim 1 or 2, wherein an impact absorbing gel piece is disposed between the cover and the artificial gluteal block.

4. The diaper unit of claim 1, wherein a concave portion is formed in a part of the artificial gluteal block coming into contact with the sacrum/coccyx such that a downslope is formed toward the outside.

5. The diaper unit of claim 1, wherein a downslope is formed toward the outside in a part of the artificial gluteal block coming into contact with a boundary portion between the gluteal region and legs.

6. The diaper unit of claim 1, wherein foamed resin is disposed in a part of the artificial gluteal block coming into contact with gluteal muscle, wherein the foamed resin has elasticity different from a member making up a major portion of the artificial gluteal block and has a shape convexed toward gluteal muscle.

7. The diaper unit of claim 1, wherein the fecal bag is made up of an inner bag containing a high-molecular absorbing polymer and a moisture-permeable waterproof outer bag.

8. The diaper unit of claim 7, wherein an opening of the outer bag of the fecal bag is coupled to a peripheral edge of the opening in the main diaper body portion by thermocompression bonding or adhesive bonding.

9. An outer diaper wrapper comprising:
an artificial gluteal block having a hole located substantially in the center, the artificial gluteal block being made up of an elastic member coming into contact with a gluteal region of a person; and
a cover containing the artificial gluteal block and having a hole corresponding to the hole of the artificial gluteal block, the cover having a hook and loop fastener disposed at least on a portion of the cover facing a gluteal region of a person when being worn.

10. The outer diaper wrapper of claim 9, wherein a bottom portion of the hole of the outer diaper wrapper is closed by the cover, and wherein a hook and loop fastener is disposed on the bottom portion of the hole.

11. The outer diaper wrapper of claim 10, wherein an impact absorbing gel piece is disposed between the cover and the artificial gluteal block.
